Europäisches Patentamt

European Patent Office

Office européen des brevets

(11) Publication number: **0 511 855 A1**

# EUROPEAN PATENT APPLICATION

(21) Application number: 92303882.2

(22) Date of filing: 29.04.92

(51) Int. Cl.⁵: **C12N 15/51,** A61K 39/29, G01N 33/576, C07K 15/00

(30) Priority: 29.04.91 US 692922

(43) Date of publication of application: 04.11.92 Bulletin 92/45

(84) Designated Contracting States: CH DE FR GB IT LI NL

(71) Applicant: MERCK & CO. INC. 126, East Lincoln Avenue P.O. Box 2000 Rahway New Jersey 07065-0900 (US)

(72) Inventor: Kniskern, Peter J. 841 Patterson Drive Lansdale, PA 19446 (US) Inventor: Hagopian, Arpi 771 Hartley Drive Lansdale, PA 19446 (US)

(74) Representative: Thompson, John Dr. et al Merck & Co., Inc. European Patent Department Terlings Park Eastwick Road Harlow, Essex CM20 2QR (GB)

(54) **HBsAg escape mutant vaccine.**

(57) In order to produce serologic variants of hepatitis B virus (HBV) surface proteins in the form of particles with substantially reduced entrapped carbohydrate content, DNA encoding the HBV variant envelope proteins was expressed in a recombinant yeast hosts which are either wild type for, or deficient in their ability to glycosylate proteins. These HBV surface proteins display the variant antigenic sites genetically encoded by the S domain of the HBV virion envelope open reading frame and contain substantially reduced levels of entrapped carbohydrate when compared with HBsAg particles produced in "wild-type" yeast cells. These particles are useful as a vaccine for both the active and passive treatment or prevention of disease and/or infection caused by HBV or other agents serologically related to HBV and as diagnostic reagents and immunogens and antigens for developing diagnostic reagents.

FIG. 1

EP 0 511 855 A1

## BACKGROUND OF THE INVENTION

Hepatitis B virus (HBV) is the infectious agent responsible for several varieties of human liver disease. Many individuals who are infected by HBV suffer through an acute phase of disease, which is followed by recovery. However, a percentage of infected individuals fail to clear their infection, thereby becoming chronic carriers of the infection. HBV infection is endemic in many parts of the world, with a high incidence of infection occurring perinatally from chronically infected mothers to their newborns who themselves often remain chronically infected. The number worldwide has been estimated at over three hundred million. From this pool of carriers, hundreds of thousands die annually from the long-term consequences of chronic hepatitis B (cirrhosis and/or hepatocellular carcinoma).

The hepatitis B delta virus is an agent which, during coinfection with HBV, is responsible for an acute fulminating disease with a generally fatal resolution. The delta virus does not encode (from its own genetic material) proteins which serve as the virion envelope; rather, the virus encapsidates with the envelope proteins encoded by the coinfecting HBV, thereby sharing a close structural and immunologic relationship with the HBV proteins (and perhaps with their variants) which are described below. It is unknown at this time whether other infectious agents share similar relationships with HBV. However, it is clear that proteins with expanded breadth of serologic re- activity or enhanced immunogenic potency would be useful in systems for diagnosis or prevention (or treatment) of diseases (or infections) by a class of agents with even slight or partial antigenic cross-reactivity with HBV.

In addition, variant HBV have been reported to replicate in the presence of vaccine-induced immunity thereby causing disease in vaccinated individuals. These variant HBV may have mutations which cause alterations in the major antigenic and protective epitopes of the HBsAg or other envelope polypeptides. Such an alteration may modify the epitope so that vaccine-induced neutralizing antibodies no longer bind [Carman, W. et al., l990, Lancet, 336, pp-325-329].

The HB virion is composed of two groups of structural proteins, the core proteins and the envelope or surface proteins. In addition to being the major surface proteins or the virion, i.e., Dane particle, the envelope proteins also are the major constituents of Australia antigen, or 22 nm particles. These envelope proteins are the translational products of the large viral open reading frame (ORF) encoding at least 389 amino acids (aa). This ORF is demarcated into three domains, each of which begins with an ATG codon that is capable of functioning as a translational initiation site in vivo. These domains are referred to as preSl (l08 aa),.preS2 (55 aa), and S (226 aa) in their respective 5′-3′ order in the gene. Thus, these domains define three polypeptides referred to as S or HBsAg (226 aa), preS2+S (28l aa), and preSl+preS2+S (389 aa), also referred to as p24/gp27, p30/gp33/gp36 and p39/gp42 respectively (as well as the major, middle and large proteins).

The envelope proteins of HBV are glycoproteins with carbohydrate side chains (glycans) attached by N-glycosidic linkages to defined peptide recognition sites. [Heermann et al., J. Virol. 52, 396 (l984) and Stibbe et al., J. Virol. 46, 626 (l983)]. Thus, the HBV polypeptides produced during natural infection comprise the species p24/gp27 (the S polypeptide and its glycosylated derivative), gp33/gp36 (the preS2+S polypeptide glycosylated in the preS2 domain only and the same polypeptide glycosylated in the S as well as the preS2 domain), and p39/gp42 (the preSl+preS2+S peptide and its derivative glycosylated in the presl domain). Currently available plasma-derived vaccines are composed of proteins containing virtually only the S domain (comprising the p24 monomer and its glycosylated derivative gp27), while yeast-derived vaccines successfully developed to date are composed exclusively of the S polypeptide (comprising exclusively the nonglycosylated p24 species).

The 22 nm HBsAg particles, have been purified from the plasma of chronic carriers. In terms of their plasma being particle-positive, these chronic carriers are referred to as HBs[+]. if infected persons have mounted a sufficient immune response, they can clear the infection and become HBs[−]. In terms of their formation of antibodies to HBs, these individuals are denoted anti-HBs[+]. In this way, anti HBs[+] is correlated with recovery from disease and with immunity to reinfection from disease and with immunity to reinfection with HBV. Therefore, the stimulation or formation of anti-HBs by HB vaccines has been expected to confer protection against HBV infection.

This hypothesis has been testable experimentally. Outside of man, the chimpanzee is one of the few species which is fully susceptible to HBV infection , as reflected in quantifiable markers such as HBs[+] and elevated serum levels of liver enzyme. Chimpanzees have been vaccinated with three doses of purified HBsAg particles and then challenged intravenously with infectious HBV. While mock-vaccinated animals have shown signs of acute HBV infection, the HBsAg-vaccinated animals have been protected completely from signs of infection. Therefore, in this experimental system, HBsAg particles, composed of p24 (or p24 and gp27), have been sufficient to induce protective immunity. Spurred by these observations, several manufacturers have produced HB vaccines composed of HBsAg particles.

The appropriate display of epitopes that elicit the production of anti-HBs is a key property associated with the excellent immunogencity of yeast-derived hepatitis B vaccines. Most important among these are the a epitopes, which are conformational determinants present on all subtypes of HBV (ayr, ayw, adr, adw) and which map to amino acids l24-l47 of the S polypeptide. A monoclonal antibody (MAb) directed against one specific "a" eptiope was shown to be capable of neutralizing the infectivity of HBV for chimpanzees [Tabor, E. et al., l983, J. Infect. Dis., l47, pp53l]. Anti-a antibodies are the most abundant elicited by HBsAg and their induction is associated with protection from disease caused by all subtypes.

Antigenic subtypes of HBV are defined serologically and have been shown to be due to single base changes in the region of the genome encoding HBsAg. Thus, major antigenic variation can result from minor changes to the genome. These subtypes occur naturally and have particular geographical distributions.

Cyclic synthetic peptides of this region have greater antigenicity than that of the linear peptides, and antigenicity of HBsAg is lost if treated with detergents [Vyas, G. et al., l972, Science, l78, ppl300-l]; these findingsimply that the epitopes are conformational. It has been shown by in-vitro mutagenesis, in which single amino acid changes can be introduced into the HBsAg molecule, that the cysteine at position l47 and the proline at position l42 are very important for the display of full antigenicity of this region [Ashton-Richardt, P. et al., l989, J. Med. Virol., 29, ppl96-203].

The HBV vaccine, which consists of HBsAg either in the native or recombinant form, is known to be highly effective. Although in about 5% of vaccinees an adequate anti-HBs response does not develop, those who do have such a response are considered protected. In persons with an adequate anti-HBs level HBV infection does not develop on subsequent exposure. It is therfore of interest that several vaccinees from southern Italy (and possibly other parts of the world), all of whom were considered to have responded well to the vaccine, whould subsequently have markers of HBV replication.

Viruses continually change during the course of infection. Patients with chronic HBV infection may have a variety of genotypes in their serum. The fact that the immune response is directed against a number of viral epitopes (some of which are neutralizing) leads to mutants being selected from the constantly changing viral population. Not all such mutants will be able to replicate, however. When an epitope is also vital to virus survival, such as when it is necessary for cell entry, a virus mutated at this position may not be viable. Replicating, infectious viruses that have mutated so that they are no longer susceptible to neutralizing immunity are termed escape mutants; it is readily apparent however that such mutants arise in non-vaccinated individuals albeit at a lower detectable frequency due to the absence of selection by antibody. Such mutation might be responsible for the antigenic drift seen regularly in the epidemiology of influenza virus, for example.

An escape mutant was identified [Carman, W. et al., supra] with an amino acid substitution of arginine for glycine at position l45 of HBsAg, and the binding of monoclonal antibodies specific to this region is much reduced. The reduction in binding of antibodies to the region of amino acids l24-l37 suggests that the mutation has resulted in a conformational change affecting several epitopes in the region of the a determinant. The extent of these mutations and whether other similar but not identical mutants exist is at present unknown.

In order to expand the available supply of HB vaccines, manufacturers have turned to recombinant DNA technology to mediate the expression of viral envelope proteins. Among microbial systems, Escherichia coli and S. cerevisiae have been used most commonly for the expression of many recombinant-derived proteins. Numerous attempts to express immunologically active HBsAg particles in E. coli have been unsuccessful. However, S. cerevisiae has shown great versatility in its ability to express immunologically active HBsAg particles. These particles (composed exclusively of p24), when formulated into a vaccine, have proven capable of fully protecting chimpanzees against challenge with live HBV of diverse serotypes. Furthermore, yeast-derived S particles are also immunologically active and as effective in preventing disease or infection in human clinical trials as plasma-derived HBsAg [Stevens et al., JAMA, 257:26l2-26l6 (l987)]. Therefore, the utility of S. cerevisiae as a host species for directing the synthesis of recombinant HBsAg is established firmly. In addition, expression of human therapeutic agents and vaccines in yeast can be very useful for product development, since yeast is free of endotoxin, is nonpathogenic to man, can be fermented to industrial scale, and lacks many of the safety concerns which surround the use of continuous mammalian cell lines (many of which are virally transformed, may be tumorigenic in mice and all of which contain protooncogenes).

S. cerevisiae (bakers' yeast) is a eukaryote which is capable of synthesizing glycoproteins. Protein glycosylation in yeast has been the subject of numerous recent review articles [notably: Kukuruzinska et al., Ann. Rev. Biochem., (l987) 56, 9l5-44; Tannen et al., BBA, (l987) 906, 8l-99]. This glycosylation or additiion of glycans to appropriate receptor amino acids (aa) on the polypeptide occurs either at specific serine (Ser) or threonine (Thr) residues (O-linked) or at specified asparagine (Asn) residues (N-linked). The specificity for O-linked addition at Ser or Thr residues is not clearly understood and is determined empirically on a case-by-case basis.

The signal sequence for N-linked glycosylation is well defined as either of the amino acid sequences Asn-X-Thr or Asn-X-Ser (wherein X is any amino acid). In addition to synthesizing many autologous, native, glycosylated proteins (among them being those called mannoproteins, or mannopeptides), yeast also are capable of glycosylating heterologous or foreign proteins expressed by recombinant technology (if the heterologous protein contains the appropriate glycosylation signal sequence for either N-linked or O-linked glycosylation).

The preS2+S polypeptides, which are produced during natural infection contain no more than two "core" [ca. 3 kilodaltons (kD) in size] N-linked glycans, one in the S region and a second on the Asn at amino acid residue 4 of the preS2 domain. The recognition site in the S domain is not glycosylated in either Recombivax HB® or in recombinant preS2+S synthesized in yeast. However, the site at amino acid residue 4 of the preS2 domain is recognized and glycosylated by yeast.

The presl domain contains an N-linked glycosylation site at amino acid residue 4 of the preSl region and a potential site at aa residue 26 for serotype adw. It is readily apparent to those skilled in the art that arguments set forth for preS2 glycosylation also will follow for diverse sequences in the preS2 region as well as for those in the presl and S domains.

Yeast synthesizing recombinant preS2+S add a "core" glycan which is similar to that added to the native polypeptide during viral infection. However, if the yeast host cell is "wild-type" for glycosylation containing the full complement of enzymes required for native glycosylation which is the case for virtually all commonly used yeast strains), a significant number of these glycans are extended with a large number of additional mannose residues in a manner identical to that employed by yeast in making its own structural mannoproteins. This extended addition of the glycan, when it occurs on a foreign gene product such as the preS2+S polypeptide, is referred to as hyperglycosylation. It is readily apparent to those skilled in the art that arguments set forth for yeast also will extend to other host cells (e.g. insect, fungi, etc.) which may be subject to divergent glycosylation patters.

Furthermore, it has been demonstrated that recombinant forms of 22nm particles of HBV surface proteins expressed in wild-type yeast host cells, entrap substantial amounts of yeast cell carbohydrate (deriving at least in part from the structural mannopeptides of the yeast host cell) within the 22nm particle. This entrapped carbohydrate could pose potential problems in that the entrapped carbohydrate may cause the generation of antibodies against yeast carbohydrate moieties on glycosylated proteins, and a vaccine immunogen containing entrapped yeast carbohydrate would react with anti-yeast antibodies present in most mammalian species thereby potentially diminishing its effectiveness as an immunogen and vaccine.

Hyperglycosylation and entrappment of complete mannoproteins and mannopeptides may be eliminated or glycosylation limited in HBV preS and S polypeptides and their corresponding particles by any of the following approaches.

Firstly, N-linked hyperglycosylation may be prevented or limited during growth of the recombinant host through the presence in the growth medium of an exogenous agent (e.g., tunicamycin). Secondly, polypeptides, from recombinant or natural sources may be deglycosylated either chemically anhydrous trifluoromethane- sulfonic acid or anhydrous hydrogen fluoride) or enzymatically with N-glycanase, Endo-F or Endo-H) or physically (e.g. sonication). Thirdly, the recognition site for glycosylation may be changed or deleted by mutagenesis at the DNA level, such that core glycosylation, and thereby hyperglycosylation as well, is prevented. Such modified preS+S ORF's in which the glycosylation recognition sequence has been altered (directed by suitable promoters active in yeast) have been transformed into yeast host cells. The resultant preS+S polypeptides lack glycosylation. Fourthly, host cells may be identified which lack critical enzymes required for glycosylation, which illustrates the present invention without however limiting the same thereto. One such yeast strain has been identified (mnn9- mutant) (Ballou, L et al., (1980), J.Biol. Chem., 255, pp 5986-599l] which lacks a critical enzyme in the glycosylation pathway necessary for the elongation (hyperglycosylation) of the N-linked glycans; chemical studies indicate that this mutant makes mannoproteins without outer-chain mannose residues and containing only the "core" carbohydrate [Ballou, C.E. et al., (1986), Proc.Natl.Acad.Sci.U.S.A., 83, pp 308l-3085; Tsai, P. et al., (1984), J.Biol.Chem., 259, pp 3805-38ll]. The ORF for the S or preS+S polypeptide (transcription directed by suitable promoters active in yeast) has been used to transform such mnn9- mutant yeast. The resulting preS+S polypeptide contains only "core" glycosylation and lacks hyperglycosylation.

Although the S polypeptides are neither glycosylated nor hyperglycosylated when expressed in yeast, particles composed therefrom contain significant levels of entrapped carbohydrate deriving from yeast mannopeptide. Therefore, the expression of S polypeptides as well as pres containing polypeptides in yeast cells which cannot hyperglycosylate results in decreased levels of carbohydrate in the expressed 22 nm particles.

S. cerevisiae has shown great versatility in its ability to express immunologically active 22 nm particles. These particles, when formulated into a vaccine, have proven capable of fully protecting chimpanzees against challenge with live HBV. Furthermore, yeast-derived HBsAg has been effective immunologically in human clinical trails as plasma-derived HBsAg. Therefore, the utility of S. cerevisiae as a host species for directing synthesis of recombinant HBsAg is established firmly.

In a variety of recombinant microbial expression systems, the synthesis of many different polpeptides has been shown to be deleterious to the host cell. As a consequence, there is selective pressure against the expression of such polypeptides, such that the only cells which accumulate in a scale-up of such a recombinant culture are.those which have ceased to express the foreign polypeptide or express so little of the foreign polypeptide that the culture becomes an uneconomical source of that polypeptide. In some cases, the deleterious effect is so strong that when expression is driven by a strong constitutive promoter, newly transformed cells fail to propagate and form colonies on selective plates. These deleterious effects can be overcome by using an inducible promoter to direct the synthesis of such polypeptides. A number of inducible genes exist in S. cerevisiae. Four well-characterized inducible genetic systems are the galactose (GAL) utilization genes, the alcohol dehydrogenase 2 (ADH2) gene, the alpha mating factor gene, and the pho5 gene.

S. cerevisiae has 5 genes which encode the enzymes responsible for the utilization of galactose as a carbon source for growth. The GALl, GAL2, GAL5, GAL7 and GALl0 genes respectively encode galactokinase, galactose permease, the major isozyme of phosphoglucomutase, α-D-galactose-l-phosphate uridyltransferase and uridine diphospho-galactose-4-epimerase. In the absence of galactose, very little expression of these enzymes is detected. If cells are grown on glucose and then galactose is added to the culture, these three enzymes are induced coordinately, by at least l,000-fold, (except for GAL5, which is induced to about 5 fold) at the level of RNA transcription. The GALl GAL2, GAL5, GAL7 and GALl0 genes have been molecularly cloned and sequenced. The regulatory and promoter sequences to the 5′ sides of the respective coding regions have been placed adjacent to the coding regions of the lacZ gene. These experiments have defined those promoter and regulatory sequences which are necessary and sufficient for galactose induction.

S. cerevisiae also has 3 genes, each of which encodes an isozyme of ADH. One of these enzymes, ADHII, is responsible for the ability of S. cerevisiae to utilize ethanol as a carbon source during oxidative growth. Expression of the ADH2 gene, which encodes the ADHII isozyme, is catabolite-repressed by glucose, such that there is virtually no transcription of the ADH2 gene during fermentative growth in the presence of glucose levels of 0.l% (w/v). Upon glucose depletion and in the presence of non-repressing carbon sources, transcription of the ADH2 gene is induced l00- to l000-fold. This gene has been molecularly cloned and sequenced, and those regulatory and promoter sequences which are necessary and sufficient for derepression of transcription have been defined.

Alpha mating factor is a sex pheromone of S. cerevisiae which is required for mating between MATα and MATa cells. This tridecapeptide is expressed as a prepropheromone which is directed into the rough endoplasmic reticulum, glycosylated and proteolytically processed to its final mature form which is secreted from cells. This biochemical pathway has been exploited as an expression strategy for foreign polypeptides. The alpha mating factor gene has been molecularly cloned and its promoter with pre-pro-leader sequence has been utilized to express and secrete a variety of polypeptides. Likewise, the pho5 gene promoter has been shown to be inducible by low phosphate concentrations and this also has utility for physiologically regulated expression of foreign proteins in yeast.

As expected by their traversal of the rough endoplasmic reticulum and Golgi apparatus, foreign proteins can undergo both N- and 0-linked glycosylation events. The alpha mating factor promoter is active only in cells which are phenotypically α. There are 4 genetic loci in S. cerevisiae, known as SIR, which synthesize proteins required for the repression of other normally silent copies of a and α information.

Temperature-sensitive (ts) lesions which interfere with this repression event exist in the gene product of at least one of these loci. In this mutant, growth at 35°C abrogates repression, resulting in cells phenotypically a/α in which the alpha mating factor promoter is inactive. Upon temperature shift to 23°C, the cells phenotypically revert to α such that the promoter becomes active. The use of strains with a ts SIR lesion has been demonstrated for the controlled expression of several foreign polypeptides.

## OBJECTS OF THE INVENTION

It is an object of the present invention to provide variant HBV surface proteins which form particles with substantially reduced entrapped carbohydrate content. It is another object of the present invention to provide a method of producing in a yeast host, variant HBV surface proteins which form particles and which also may contain substantially reduced entrapped carbohydrate content. An additional object of the present invention is to provide a vaccine against variant HBV comprising the variant HBV surface protein particles with substantially reduced entrapped carbohydrate content for both active and passive treatment of prevention of disease and/or infections caused by variant HBV or other agents serologically related to HBV, and to provide antigens and immunogens for development of diagnostic reagents for such diseases and/or infections. A further object of the present invention is to provide conditions for the large scale growth of recombinant host cells and the purification of the recombinant variant HBV surface proteins. These and other objects of the present invention will be apparent from the following description.

## SUMMARY OF THE INVENTION

Variant HBV surface proteins have been expressed at high yield in recombinant yeast hosts which are wild type for or genetically deficient in the ability to glycosylate proteins. The expression of variant HBV surface proteins in yeast cells results in the formation of the characteristic HBsAg particles. Formation of these particles in yeast cells results in the entrapment of yeast cell substances within the particles. Using "wild-type" yeast host cells substantial amounts of yeast cell carbohydrate may become entrapped within the HBsAg particles. In order to circumvent the production of a HBV vaccine consisting of particles which contain substantial amounts of carbohydrate, the HBV surface proteins were produced and purified from a recombinant yeast host which is genetically deficient in its ability to glycosylate proteins. The variant HBV surface proteins produced by such a host form particles which contain substantially less carbohydrate than particles produced in wild-type yeast cells. These variant HBV surface proteins are useful as a vaccine for the treatment and/or prevention of HBV related infections, and as an antigen for immunologic diagnosis with reduced reactivity with naturally occuring anti-yeast antibodies, and improved reactivity to variant viruses.

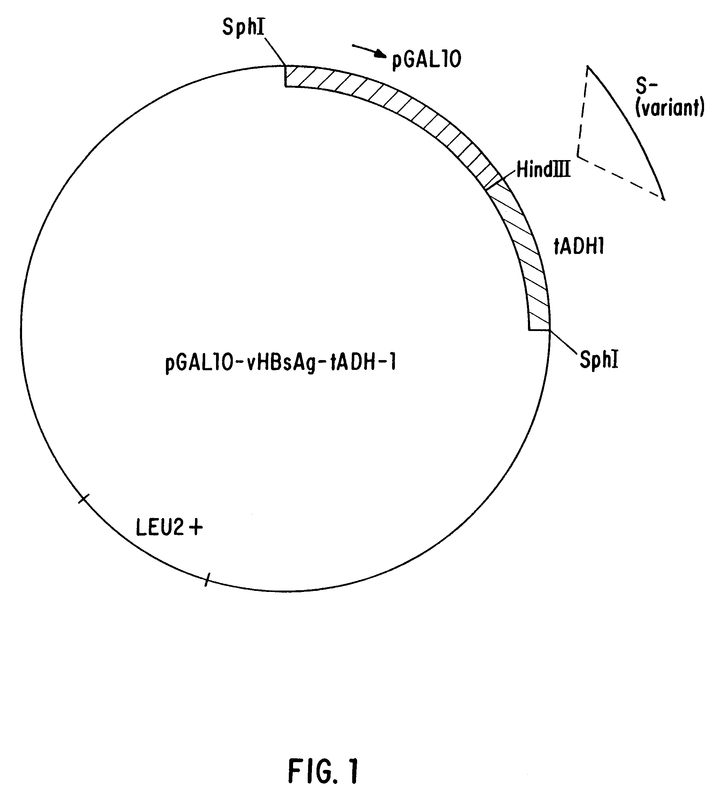

## BRIEF DESCRIPTION OF THE DRAWING

Figure I is a schematic diagram of plasmid pGALI0-vHBsAg-tADHI which contains the GALI0 promoter, the unique HindIII site for cloning of the variant HBsAg coding region, and the tADH-I terminator.

## DETAILED DESCRIPTION OF THE INVENTION

The present invention is directed to a method for the preparation of antigenic variant HBV surface protein particles which may contain substantially reduced amounts of entrapped carbohydrate, for use as a vaccine or diagnostic against HBV.

Dane particles (serotype adw) were utilized as the source of HBV nucleic acid for the isolation of the viral ORFs. It is readily apparent to those skilled in the art that this invention extends to the use of nucleic acid from HBV strains or related viruses with other serologic reactivities which derive from viral genetic diversity. The endogenous polymerase reaction was employed in order to produce covalently-closed circular double-stranded DNA of the HBV genome from the nicked and gapped nucleic acid form that natively resides in the HB virion. The DNA was isolated, digested to completion with EcoRI, and cloned into the EcoRI site of pBR322, thus generating pHBV/ADW-I. The recombinant plasmids containing the HBV genome in a circularly permuted form at the EcoRI site of the PreS region were selected. The complete ORF encoding the 55 amino acids of the preS2 region, and the 226 amino acids of the S region was constructed first by purifying the 0.8 kilobase pair (kbp) fragment obtained following digestion of pHBV/ADW-I with EcoRI and AccI; this fragment encodes the preS2+S polypeptide lacking only the initiation codon, the amino-terminal 3 amino acids, the carboxy-terminal 3 amino acids, and the translational terminator codon.

Oligonucleotides were synthesized and ligated to this fragment, converting it to a HindIII fragment containing a I0 bp yeast-derived non-translated 5′ flanking sequence and the complete preS2+S ORF was chosen such that the termination codon was directly joined to a natural HindIII site in the ADHI transcriptional terminator, thus creating a completely native yeast-derived junction without any additional intervening bases. It is readily apparent to those skilled in the art that for expression of HBV surface and related proteins, any suitable yeast-active transcriptional terminator may be substituted for ADHI.

The 5′ flanking sequence for the construction:

$$\text{ACAAAACAAA (SEQIDNO: 1)}$$
$$1 \qquad 10$$

was chosen to correspond to that for the non-translated leader (NTL) of the yeast gene GAP63 (GAP) [Holland, J.Biol. Chem., 225, 2596 (I980)] and is also a consensus for the GAP gene family. The construction was made in such manner as to join the NTL directly to the initiation codon of the preS2+S ORF without the intervention of any additional bases. Therefore, it is readily apparent to those skilled in the art that, for expression of HBV surface proteins, the selection of NTL sequences extends to other sequences which result in suitable expression levels.

DNA sequence analysis revealed 2 base substitutions which resulted in amino acid differences from the preS2+S sequence encoded by the DNA of pHBpreSGAP347/I9T [Valenzuela et al., Biotechnology, 3(4), 3I7-320 (I985)]. In order to evaluate indentical polypeptides for both constructions, these nucleotide substitutions, which were T instead of C at base 64 of the 846 bp ORF of HBV preS2+S (encoding Phe rather than Leu) and C instead of A at base 352 (encoding His rather than Gln) were changed by site-directed mutagenesis [Zoller et al., Nucleic Acids Research l0:6487-6500 (1982)]. The encoded amino acid sequence for the optimized construction then was verified. It is readily apparent to those skilled in the art that this invention is not limited to this sequence and extends to any sequence wherein the DNA encodes a polypeptides with HBV-related antigenicity.

The large DNA fragment of 3.3kbp which contains pUCl9 and the HBsAg coding region was separated from the preS2 encoding DNA fragment after digestion with EcoRI and StyI, and purified by preparative agarose gel electrophoresis. A synthetic DNA oligonucleotide was then ligated with the pUCl9-HBsAg fragment. This synthetic DNA oligonucleotide contains 5′ EcoRI and 3′ StyI sticky ends as well as providing a HindIII site immediately following the 5′ EcoRI site. In addition, the synthetic DNA oligonucleotide contains the HBsAg ATG codon plus the 9 upstream nucleotides and the 2l downstream nucleotides including the StyI site.

This oligonucleotide rebuilds the complete coding region of the HBsAg and allows its subsequent removal intact, from the pUCl9 based vector by digestion with HindIII.

The pUCl9-HBsAg DNA fragment with the ligated synthetic DNA olgonucleotide described above was used to transform E. coli. Recombinant plasmids were selected which possess the complete reconstructed HBsAg coding region. The complete HBsAg open reading frame (ORF) was removed from the recombinant plasmid by digestion with HindIII followed by isolation and purification of the 0.7kbp HBsAg DNA by preparative agarose gel electrophoresis or cloning into a GALl0 promoter expression vector.

A pUCl9 based plasmid containing the variant "a" epitope HBsAg ORF without the presl or preS2 coding regions was constructed as follows. The pUCl9-HBsAg plasmid with the complete HBsAg coding region, described previously, was used as a template for polymerase chain reaction (PCR) to synthesize DNA containing a single base substitution at position 587 (numbered from the unique EcoRI site of the HBV genome) which results in an amino acid substitution from Gly to Arg at amino acid l45 of HBsAg. The complete coding region of the variant HBsAg can be removed intact from the pUCl9 based vector by digestion with HindIII.

The pUCl9-variant HBsAg plasmid was used to transform E. coli. Recombinant plasmids were selected which possess the complete variant HBsAg coding region. The complete variant HBsAg ORF was removed from this recombinant plasmid by digestion with HindIII followed by isolation and purification of the 0.7kbp variant HBsAg DNA by preparative agarose gel electrophoresis for cloning into a GALl0 promoter expression vector.

The expression cassette (pGALl0-tADHl) drives expression of foreign genes inserted at a unique HindIII site downstream from the galactose-inducible GALl0 promoter. The HBsAg ORF (with HindIII termini) described above was ligated into the HindIII site of the vector. This expression cassette was inserted between the SphI sites of the E. coli/S. cerevisiae shuttle vector pCl/l (Beggs, supra) and this vector was introduced into S. cerevisiae strains CF52 and CF54, and transformed clones were selected.

The fragment encoding the variant S ORF described above was used to construct an expression cassette, as described previously [Kniskern et al., Gene 46:l35-l4l, (1986)], which was composed of: (a) ca. l.l kbp of the GAP49l promoter, (b) a l0 bp yeast-derived flanking sequence, (c) 68lbp of the viral ORF for S, and (d) ca. 0.4 kbp of the yeast ADHl terminator.

Three different expression vectors were used to construct HBsAg expression cassettes. The GAP 49l promoter expression cassette described previously [Kniskern et al., 1986 Gene 46 ppl35-l4l], is composed of about l.l kbp of the glyceraldehyde-3-phosphate dehydrogenase (GAPDH) promoter and about 350bp of the yeast alcohol dehydrogenase I (ADHI) terminator in a pBR322 backbone, with a unique HindIII site between the promoter and terminator. The HBsAg ORF from Example 2 was ligated in the unique HindIII site, and its presence and orientation confirmed by restriction endonuclease analyses and Southern blot.

Alternately the (0.5kbp) GALl0 promoter (Schultz et al., 1987, Gene, 54, ppll3-l23) was substituted for the l.lkbp GAP promoter in the above construction, or the (l.25 kbp) ADH2 promoter (Kniskern et al., 1988 Hepatology 8, 82-87) was substituted for the GAP promoter (see Figure l).

In each case, the expression cassette containing the specific promoter, the HBsAg ORF, and the ADHI terminator was cloned into the shuttle vector pCl/l (Beggs, supra; Rosenberg, et al., supra) to create a yeast expression vector which was then used to transform S. cerevisiae as described below. These transformants were established as frozen stocks for evaluation and subsequent experimentation. Parental strain CF52 was obtained as follows: The α mating type strain CZ5/LB347-IC (mnn9⁻, SUCZ⁻) was mated with the a type strain 2l50-2-3 (leu2⁻, adel⁻) by mixing the strains on a YEHD complete media plate. To select for diploids, the mated strains were replica plated onto leu⁻ minimal medium containing 2% sucrose as the sole carbon source. After isolating single colonies, the diploids were sporulated, and asci were dissected by standard techniques. The KHY-l07 strain was isolated as a single spore and characterized as cir⁺, adel⁺, leu2⁻, and mnn9⁻ (by Schiff stain technique).

KHYI07 (cir 0) was derived from strain KHYI07 (cir⁺) as described by Broach [Methods in Enzymology, l0l, Part C, pp 307-325, (l983)]. The cured strain was made ura3⁻ by integrating a disrupted ura3 gene. The resulting strain, KHY-l07ura3Δ, was grown in rich media to allow the accumulation of spontaneous mutations and a canavanine resistant mutant was selected. The mutant strain, CF55, was shown by complementation tests to be canl⁻. The GALl0pGAL4 expression cassette was integrated into the HIS3 gene of CF55 (Methods in Enzymology, l990, l85 pp297-309) to yield the final host strain CF52 (Mata leu2-2,ll2 ura3A canl his3Δ::GALl0pGAL4-URA3, cir°). These transformants were established as frozen stocks for evaluation and subsequent experimentation.

Recombinant yeast from the frozen stocks was grown in YEHD medium [Carty et al., J. Industrial Micro., 2, ll7-l2l, (l987)]. After growth to stationary phase, yeast cells were harvested. Lysates were prepared, resolved by sodium dodecylsulfate-polyacrylamide gel electrophoresis (SDS-PAGE), and immunoblotted with antibodies to HBsAg. One polypeptide was found with molecular weight of about 24-kD in accord with the predicted molecular weight of the translational product of the S ORF. Furthermore, lysates of recombinant, but not parental, yeast were positive for S by radioimmunoassay (Ausria^R). Electron microscopic examination of partially purified yeast lysates showed high densities of typical HBsAg particles.

The yeast-derived promoter initiates transcription of the HBsAg and related genes. Therefore, it is readily apparent to those skilled in the art that any yeast-active promoter sequence (e.g. including but not limited to GALl, GALl0, ADH2 pho5, etc.) may be substituted for the GAP49l promoter. It is also readily apparent to those skilled in the art that a suitable assay system, e.g., immunoblot or RIA or enzyme-linked immunoassay (EIA), should be utilized in order to assay expression of HBsAg and related polypeptides in this system, such that the time of harvesting of the culture for attaining a maximal yield can be optimized.

The GAP49l promoter has been useful for the expression in yeast of several foreign proteins, including HBsAg [Bitter et al., Gene, 32:263-274, (l984); Wampler et al., Proc. Nat. Acad. Sci. USA, 82:6830-6834, (l985)). Based upon our previous results of expressing HBcAg to about 40% of soluble yeast protein (Kniskern et al., supra), we have used this promoter to drive the expression of HBsAg and related proteins in suitable yeast host cells.

It is readily apparent to those skilled in the art that the selection of a suitable yeast strain for expression of HBV surface proteins encompasses a wide variety of candidates. Suitable yeast strains include but are not limited to those with genetic and phenotypic characteristics such as protease deficiencies, and altered glycosylation capabilities.

In order to control and define the glycosylation of recombinant yeast-expressed HBV proteins, S. cerevisiae strain CF52 (Mata leu2-2, ll2 ura3Δ canl his3Δ::GALl0pGAL4-ura3, cir°, mnn9-) which was constructed as described above.

The expression plasmid pCl/lpGALl0-vHBsAg-tADH-l was used to transform CF52 (Mata leu2-2, ll2 ura3Δ canl his3Δ:: GALl0pGAL4-ura3, cir°, mnn9-). Transformed clones were selected on minimal medium (leu-) containing lM sorbitol. These cloned transformants were established as frozen stocks in l7% glycerol for subsequent evaluation and further experimentation.

To provide a glycosylation wild-type control the expression plasmid was also used to transform yeast strain CF54 which was isolated by standard techniques from strain CF52 and which is a spontaneous revertant to MNN9+ (and thus is wild-type for glycosylation but otherwise is of identical genotype to strain CF52). Transformed clonal isolates were established as frozen stocks in l7% glycerol for subsequent evaluation and further experimentation.

Clones of transformed yeast containing the expression plasmids were plated onto leu⁻selective agar plates (containing lM sorbitol for the mnn9- mutants) and incubated at 30°C for 2-3 days. These yeast were inoculated into 5-7 mL cultures of complex YEHD (Carty et al., supra) medium (containing 0.l to lM sorbitol), and the cultures were incubated at 30°C with aeration for l2-l8 hours. Flasks containing 50 mL complex YEHD media with 0.lM sorbitol (hereafter called YEHDS), plus 2% galactose for the GALl0 promoter plasmids, were inoculated from the above cultures (to an inital $A_{600}$ = 0.l) and were incubated at 30°C with shaking (350 rpm) for 48-72 hours to a final $A_{600}$ of l0-l6. Samples of l0 $A_{600}$ units were dispensed into tubes, and the yeast cells were pelleted by centrifugation at 2000xg for l0 minutes. Samples either were assayed directly or stored frozen at -70°C. At the time of assay, the pellets were resuspended in 0.4 mL of phosphate- buffered saline (PBS) containing 2mM phenylmethyl sulfonyl fluoride (PMSF) and transferred to l.5 mL Eppendorf tubes. Yeast cells were broken by: l) the addition of 200-300 mg of washed glass beads (0.45 mm) and agitation on a vortex mixer for l5 minutes, 2) addition of TRITON X-l00 to 0.5%, 3) agitation on the vortex mixer for 2 minutes, and 4) incubation at 4°C for l0 minutes. Cellular debris and glass beads were removed and the supernatant was assayed for protein [by the method of Lowry et al., J. Biol. Chem., l93, 265, (l95l)] and S (AUSRIA^R).

Immunoblot analysis of the polypeptide derived from all recombinant clones described above, in host cells with the mnn9- phenotype, showed one band with apparent molecular size of about 24-kD.

For recombinant proteins, the qualitative and quantitative glycosylation patterns are a function of and largely dependent upon the host cell species, and within a species upon the cell line. It is thus readily apparent to those skilled in the art that the selection of a host strain extends to species and cell lines other than <u>S</u>. <u>cerevisiae</u> for which mutations in enzymes in the glycosylation pathway may be identified. It is also readily apparent to those skilled in the art that selection of host strains of <u>S</u>. <u>cerevisiae</u> extends to all strains in which mutations in enzymes of the glycosylation pathway may be identifed.

The transformed clones were then screened for the presence of the pUCl9-HBsAg DNA and expression of p24 HBsAg. Cells were grown in YEHDS medium also containing galactose for the GALl0 promoter plasmids, to induce expression following glucose depletion. Lysates were prepared, resolved by sodium dodecyl sulfate-polyacrylamide gel electrophoresis (SDS-PAGE) and Western blotted to nitrocellulose. A p24 product was found to be specific to S protein by virtue of its presence only in induced transformants and its reactivity with anti-p24 serum. One of these was selected for further analysis. Furthermore, lysates of transformants, but no parental <u>S</u>. <u>cerevisiae</u>, were positive for HBsAg by radioimmunoassay.

This highlights the utility of the expression vector which utilizes the <u>GALl0</u> promoter to direct the expression of HBsAg and related surface proteins in <u>S</u>. <u>cerevisiae</u>. It is readily apparent to those skilled in the art that the regulatable <u>GALl0</u> promoter, or <u>GALl</u>, <u>GAL2</u>, <u>GAL7</u> or <u>MELl</u> promoters which function in an indistinguishable manner, enable the growth of a recombinant <u>S</u>. <u>cerevisiae</u> culture to be scaled up to a production-scale volume before synthesis of the recombinant protein is initiated, such that negative effects on the host cell are minimized. Moreover, it is readily apparent to those skilled in the art that an expression vector containing another regulatory promoter, including but not limited to <u>ADH2</u> and alpha mating factor, physiologically inducible or derepressible by other means, can be utilized to direct expression of S and preS-containing peptides. Furthermore, it is readily apparent to those skilled in the art that a constitutive promoter less potent than <u>GAPDH</u>, including but not limited to <u>CYCl</u>, drives expression of S and pre-S-containing polypeptides to a lower percentage of cell protein, such that the negative physiological effects of overexpression would be obviated. It is readily apparent to those skilled in the art that a suitable assay system, <u>e.g.</u> Western blot or radioimmunoassay, should be utilized in order to assay expression of S and pre-S-containing polypeptides in this system so that the time of harvesting of the culture for attaining a maximal yield can be optimized.

An immune-affinity column, bound with goat antibodies which recognize the particulate form of HBsAg, has been utilized to purify S and S-related proteins from transformed <u>S</u>. <u>cerevisiae</u>. The eluted product is positive for HBsAg by radioimmunoassay, and is of particulate form in electron microscopy. Such a particulate form which contains both HBsAg and pre-S antigens or HBsAg alone is effective as a HBV vaccine and diagnostic reagent.

Yeast cells transformed with expression vectors coding for a hepatitis B virus surface protein or variants thereof are grown and harvested. The cells may be stored if desired by washing the cells in a buffer solution, <u>e.g.</u> PBS, and forming a cell paste which is typically stored frozen at -70°C.

Purification of HBsAg and related proteins typically begins as follows. A batch of fresh or frozen cell paste is suspended in a buffer, preferably TRIS, at a high pH ranging between about 8.5 and about ll.0, preferably about l0.5 (the buffer may also contain suitable protease inhibitors). The cells are then disrupted, preferably by mechanical means. The gentle bead breakage method of disruption has been found to be unsuitable for scale-up use. Disruption by a high pressure homogenizer (about l0,000 to 20,000psi, using a Gaulin or Stansted homogenizer) is preferred because of its rapid and efficient operation.

Disruption of the yeast cells results in a crude extract. The crude extract is then pH adjusted. The pH is adjusted to within the range of 8.0 to ll.0, with l0.5 being preferred.

It may be desired at this point to add a detergent to the crude extract. The addition of a detergent will facilitate the separation of yeast cell membranes from unwanted cellular debris. It has been shown that preS2+S protein, as well as other forms of the surface proteins, may associate with yeast cell membranes. A variety of neutral or non-ionic detergents can be used, including but not limited to detergents of the TRITON-N series, TRITON-X series, BRIJ series, TWEEN series or EMASOL series, deoxycholate, octylglucopyranoside or NONIDET-NP-40. Zwitterionic detergents such as CHAPS or CHAPSO are also useful and suitable agents.

If a detergent is to be used, the preferred detergent is TRITON X-l00 at a concentration of about 0.5%. It must be stressed that the method of this invention does not require detergent use at this step and the use of detergents is optional.

The extract then may be heat treated if protease inhibitors were not added during lysis. Heat treatment is effective over a range of temperatures and for a range of treatment duration. Typically a temperature range of 45°C to 60°C is used, with 50°C as the preferred temperature. The duration of heat treatment typically ranges between 20 to 40 minutes with 30 minutes as the preferred time. The extract is heat treated in a suitable vessel and the vessel is immersed in a heated bath, or a heat exchanger is used. The material is then cooled to about l0°C, preferably by placing it into an ice-water bath or by using a heat exchanger. It will be readily apparent to those skilled in the art that, according to the method of this invention, the order in which the heat treatment and the debris removal steps are done may be reversed without significant effect on the result of this procedure. Alternatively, whole yeast cells can be heated in a neutral pH buffer, disrupted and detergent added as described above.

Removal of cellular debris from the heat treated crude extract is necessary to prevent physical occlusion during subsequent purification steps. Debris can be removed by centrifugation, microfiltration, or filtration producing a clarified extract. Centrifugation and microfiltration are the most preferred methods. Centrifugation can be done for varying lengths of time at different centrifugal forces. Centrifugation at about 3,000 x g for 15 minutes at 4°C has been found adequate. It may also be advantageous to dilute the extract before centrifugation to reduce the typically viscous nature of a crude yeast cell extract. Dilution will not alter any subsequent steps of this procedure.

Microfiltration has an advantage in that filtration and dialysis can be performed simultaneously. Several types of microfiltration units are suitable for use in this step, e.g. KROSFLO by Microgon Inc. or any variety of hollow fiber cartridges by Amicon or A/G Technology. The preferred microfiltration method is to pass the extract through Prostak Durapore (Millipore) membrane, plate and frame microfiltration unit with a pore size of about 0.1 microns to 0.2 microns, at an inlet pressure of about 2 to 7 psi, using a buffer consisting of about 0.1M TRIS, pH about 10.4 and about 0.1% TRITON X-100.

The supernatant from centrifugation or the filtrate from microfiltration may be concentrated prior to the next step of this procedure. Concentration can be achieved by several methods including, but not limited to, dialysis, filtration, lyophilization, ultrafiltration and diafiltration. The preferred method of concentration of the present invention is to run the clarified extract through a $10^5$ molecular weight cut off, hollow fiber ultrafiltration system. The volume of the clarified extract is typically reduced by about 6.5 fold for the microfiltration product and about 2 fold for the diluted, centrifuged product, yielding a concentrated retentate. Following concentration, the retentate is diafiltered to further remove lower molecular weight contaminants. Diafiltration is performed using a $10^5$ molecular weight cutoff, hollow fiber system.

If TRITON X-100 was added, it can be removed by several conventional methods including, but not limited to, dialysis, addition of certain organic solvents, refrigeration, chromatographic separation, and contact with a gel or resin which specifically binds detergents, such as Extractogel (Pierce) and XAD resin (Romicon). The preferred method of this invention to remove TRITON X-100 is to circulate the heat treated extract containing TRITON X-100 through a cartridge of XAD-2 or XAD-4 resin (polystyrene divinylbenzene). The heat treated extract is circulated through the XAD cartridge for about 10 hours at 4°C and then collected in a suitable vessel, for example, a sealable glass bottle.

If the cells were disrupted in a high pH buffer, the pH of the extract is then adjusted to between about pH 7.0 to about 7.9 with the preferred pH of about 7.7. Adjusting the pH to about 7.7 following heat treatment at a high pH according to the method of this invention, greatly facilitates the adsorption of envelope proteins to the wide pore silica utilized in a subsequent step. Adjustment of the pH of the heat treated extract can be performed prior to the Triton X-100 removal step without effecting the outcome of the procedure. Therefore, it will be obvious to those skilled in the art that, according to the method of this invention, the order in which the pH adjustment and the Triton X-100 removal steps are done may be reversed without significant effect on the result of this procedure.

The HBsAg is then easily separated from the contaminants yielding substantially purified HBsAg. The preferred method of eliminating the contaminants is to adsorb the HBsAg onto wide pore silica. The most preferred method of this invention is to adsorb the HBsAg onto a wide pore silica with a pore size range of about 1000 to 1500 angstroms and silica particle size range of about 30 to 130 microns (Amicon). The HBsAg readily enters the pores of the silica and is retained. The yeast cellular protein contaminants can therefore be easily washed away.

Adsorption of HBsAg onto wide pore silica can be done chromatographically or in a non-chromatographic, batchwise fashion. Chromatographic adsorption is done by passing the pH adjusted extract through a bed of wide pore silica in a column chromatography apparatus. Typically, about one liter of heat treated extract is applied to a 5 cm jacketted column apparatus containing about 300 ml (about 100 g dry weight) of wide pore silica beads at a flow rate of about 200ml/hour.

Non-chromatographic adsorption onto wide pore silica is typically done by mixing the heat treated extract with the silica in a suitable vessel, e.g. a sealable glass bottle. The preferred method is to add 300 ml of wide pore silica to about one liter of heat treated extract in a glass bottle and incubate with constant mixing. Adsorption preferrably continues for about 1.5 hours at about 4 - 8°C although different times and temperatures are suitable.

Washing of the surface protein-adsorbed silica free of unadsorbed material can also be done non-chromatographically, or the silica can be poured into a column apparatus, as previously described, for chromatographic adsorption. Batchwise washing is done by draining the heat treated extract from the wide pore silica and adding several volumes of a buffer which will not cause the release of HBsAg adsorbed onto the silica. The preferred buffer is PBS. The silica is drained and the washing steps are repeated 3 to 5 times.

Chromatographic washing of the HBsAg-adsorbed silica is done by passing PBS through the silica at a flow rate of about 200ml/hour until the extinction at 280nm is constant.

The HBsAg is eluted from the washed wide pore silica using a buffer solution with a pH between about 8.5 to 9.0. Surface proteins are preferably desorbed using a buffer solution consisting of about 0.05 M Borate at a pH of about 8.7. Desorption of HBsAg can be facilitated at elevated temperatures over a wide range. Desorption at about 55°C is preferred.

Non-chromatographic desorption is done by mixing 1200 ml of 0.05 M Borate buffer at pH 8.7 with about 700 ml of washed HBsAg-adsorbed wide pore silica. Desorption continues for about 25 minutes. The eluate is then collected, the desorption steps are repeated twice and the eluate is cooled.

Chromatographic desorption is done by warming the jacketted column of washed silica to about 55°C. The 0.05M Borate buffer at pH 8.7 is warmed to 55°C and then applied to the column at a rate of 500 ml/hour. The eluate is then collected and cooled. The volume of eluate is usually roughly equivalent to the volume of heat treated extract applied to the wide pore silica.

Concentration of the eluted HBsAg is usually desired. The preferred concentration method is to pass the eluate through a $10^5$ molecular weight cut-off hollow fiber diafiltration system using a 0.05M Borate buffer, pH 8.7. The volume of the eluted surface protein may be generally reduced by as much as 16 fold using this system. The diafiltration retentate can be sterilized by microfiltration if necessary.

The carbohydrate content of the HBsAg is determined by the method of Dubois, M. et al., Anal. Chem., 28, pp.350, 1956. The general principle of this procedure is that simple sugars, oligosaccharides, polysaccharides and their derivatives, including the methyl ethers with free or potentially free reducing groups, give an orange yellow color when treated with phenol and concentrated sulfuric acid. The amount of color produced at a constant phenol concentration is proportional to the amount of sugar present.

To determine the carbohydrate content of a sample of HBV surface proteins, I mL of a solution containing between 10 to 70 μg of protein is placed in a test tube. A series of carbohydrate standards and blank samples are prepared. One mL of a 5% phenol solution is added to each tube, the tubes are mixed, and 5 mL of a 96% sulfuric acid solution is added and mixed. The tubes are incubated at room temperature for 10 minutes, mixed, and incubated at 25 to 30°C for 20 minutes. The samples are read in spectrophotometer ($A_{490}$ for hexoses and methylated hexoses, and $A_{480}$ for pentoses, uronic acid, and their methylated derivatives) and the amount of carbohydrate in the HBsAg samples is determined by comparison with the carbohydrate standards.

HBsAg produced in "wild-type" recombinant yeast cells (CF54), and HBsAg produced in the CF52 (mnn9-) recombinant yeast cells were both analyzed for carbohydrate content as described above. Based on these results, a ratio of the amount of carbohydrate to protein present in each sample was calculated by dividing the micrograms of carbohydrate by the micrograms of protein in the sample. This ratio calculation demonstrated that HBsAg produced in mnn9⁻ recombinant yeast cells consistently contained one tenth of the carbohydrate content of HBSAG produced in recombinant "wild-type" yeast cells. These results show that HBsAg produced in the mnn9⁻ mutant yeast cells contained substantially reduced amounts of carbohydrate when compared to HBsAg produced in "wild-type" yeast cells.

The following examples illustrate the present invention without, however, limiting the same thereto. The disclosure of each reference mentioned in the following examples is hereby incorporated by reference.

EXAMPLE I

Cloning of HBV DNA in pBR322

HBV Dane particles (serotype adw) were isolated and purified from human plasma (carrier), and double-stranded DNA was synthesized by the endogenous polymerase in the Dane particles according to the methods of Landers et al., [J. Virology, 23, 368-376, (1977)] and Hruska et al., [J. Virology, 21, (1977)]. The DNA was isolated after digestion with Proteinase K in SDS followed by extraction with phenol/chloroform and ethanol precipitation. The HBV genomic DNA was digested with EcoRI, producing a single 3.2 kbp fragment, that was cloned into the EcoRI site of pBR322 to form pHBV/ADW-I. The presence of the HBV DNA was confirmed by EcoRI digestion, Southern blot transfer to nitrocellulose, and hybridization with [$^{32}$p]-labelled specific oligonucleotide probes.

EXAMPLE 2

Cloning of the preS2+S Gene into the pGAP-tADH-2 Expression Vector

Plasmid pHBV/ADW-I (described in Example I) was digested with EcoRI and AccI, and the 0.8 kbp fragment was purified by preparative agarose gel electrophoresis. Also, a pUC plasmid was digested with EcoRI and BamHI and the linear vector was purified by preparative agarose gel electrophoresis.

To reconstruct the 5′ portion of the preS2+S ORF, a pair of oligonucleotides was synthesized which reconstitutes the ORF from the EcoRI site upstream to the ATG through a I0 bp NTL sequence through a HindIII site to an EcoRI terminus. The sequence of these oligonucleotides is:

```
AATTCAAGCT TACAAAACAA AATGCAGTGG (SEQIDNO: 2)
1          10         20         30


GTTCGAATGT TTTGTTTTAC GTCACCTTAA (SEQIDNO: 3)
1          10         20         30
```

To reconstitute the 3′ portion of the preS2+S ORF, a second pair of oligonucleotides was synthesized which reconstitutes the ORF from the AccI site through the translational terminator through a HindIII site to a BamHI terminus. The sequence of these oligonucleotides are:

```
ATACATTTAA AGCTTG (SEQIDNO: 4)
1          10   16


TGTAAATTTC GAACCTAG (SEQIDNO: 5)
1          10     18
```

The oligonucleotide pairs were annealled, then ligated to the pUC EcoRI - BamHI digested vector. The resultant vector (2.8kbp) was purified by preparative agarose gel electrophoresis. The 0.8kbp EcoRI - AccI fragment from above was ligated with this vector. The presence and orientation of the PreS2+S ORF was confirmed by restriction endonuclease analysis and Southern blot. DNA sequence analysis [Sanger et al., 1977] revealed two base substitutions that resulted in amino acid differences from the sequence encoded by the DNA of plasmid HBpreSGAP347/I9T. To evaluate identical polypeptides for both constructions, these substitutions, which were T instead of C at base 64 (encoding Phe rather than Leu) and C instead of A at base 352 (encoding His rather than Gln), were changed by site-directed mutagenesis. (Zoller and Smith I982, Nucleic Acids Research, I0, pp6487-6500].

A plasmid containing the HBsAg coding region without the preS2 coding region was constructed as follows: The pUCHBpreS2+S plasmid (described above) was digested with EcoRI and StyI restriction endonucleases. The large DNA fragment (3.3kbp) which contains pUC and the HBsAg coding region was separated from the preS2 encoding DNA fragment and purified by preparative agarose gel electrophoresis. A synthetic DNA oligonucleotide pair:

```
AATTCAAGCT TACAAAACAA AATGGAGAAC ATCACATCA
1          10         20         30        40
GGATTC (SEQIDNO: 6)
     45
```

```
GTTCGAATGT TTTGTTTTAC CTCTTGTAGT GTAGTCCTAA
1          10         20         30        40
GGATC (SEQIDNO: 5)
     45
```

was then ligated with the pUCHBsAg fragment. This synthetic oligonucleotide pair contains 5′ EcoRI and 3′ StyI sticky ends as well as providing a HindIII site immediately following the 5′ EcoRI site. In addition, the synthetic DNA oligonucleotide pair contains the HBsAg ATG codon, the upstream l0bp non-translated leader sequence, and the 2l downstream nucleotides including the StyI site.

This oligonucleotide pair rebuilds the complete coding region of the HBsAg and allows its subsequent removal intact, from the pUC-based vector by digestion with HindIII.

The pUC-HBsAg DNA vector with the ligated DNA oligonucleotide pair described above was used to transform E. coli. Recombinant plasmids were selected which possess the complete reconstructed HBsAg coding region. The complete HBsAg open reading frame (ORF) was removed from the recombinant plasmid by digestion with HindIII followed by isolation and purification of the (0.7kbp) HBsAg DNA by preparative agarose gel electrophoresis for cloning in to an expression vector.

EXAMPLE 3

Cloning of the HBsAg a epitope variant

A pUCl9 based plasmid containing the variant "a" epitope HBsAg ORF without the presl or preS2 coding regions was constructed as follows. The pUCl9-HBsAg plasmid with the complete HBsAg coding region, described previously, was used to prime DNA synthesis by polymerase chain reaction (PCR) using the Perkin Elmer/Cetus GeneAmp DNA Amplification Reagent Kit, and following the manufacturer's recommended procedures.

Priming was achieved with two oligonucleotides from StyI to BstXI sites. The first olidonucleotide at the BstXI site has the following sequence:

```
CCAGGACGAT GGGATGGGAA TACAGGTGCA
1          10         20         30
ATTTCGATCC (SEQIDNO: 8)
     40
```

and includes a single base change (G to C) resulting in the amino acid substition of Arg for Gly at amino acid l45 in the a epitope region of the HBsAg, was introduced into the DNA encoding HBsAg.

A second DNA oligonucleotide was synthesized with the sequence from the StyI site:

```
AGGATTCCTA GGACCCCTGC TCGTGTT (SEQIDNO: 9)
1          10         20      27
```

The oligonucleotide was used to prime DNA synthesis from the StyI site to the BstXI site of the HBsAg DNA using polymerase chain reaction (PCR) and the DNA produced from the first oligonucleotide primer containing

EP 0 511 855 A1

the G to C substitution, according to the manufacturer's procedure.

Double stranded DNA (about 450bp long) was isolated and purified by preparative agarose gel electrophoresis, digested with StyI and BstXI, and ligated into the isolated and purified pUCHBsAg vector which was digested with StyI and BstXI described above to remove the "a" epitope.

The resulting vector was confirmed to contain the modified "a" epitope by restriction endonuclease mapping and DNA sequencing.

EXAMPLE 4

Cloning of the variant HBsAg ORF into 3 different expression vectors

Three different expression vectors were used to construct variant HBsAg expression cassettes. The GAP 49I promoter expression cassette described previously [Kniskern et al., I986 Gene 46 ppI35-I4I], is composed of about I.I kbp of the glyceraldehyde- 3-phosphate dehydrogenase (GAPDH) promoter and about 350bp of the yeast alcohol dehydrogenase I (ADHI) terminator in a pBR322 backbone, with a unique HindIII site between the promoter and terminator. The HBsAg ORF from Example 2 was ligated in the unique HindIII site, and its presence and orientation was confirmed by restriction endonuclease analyses and Southern blot.

Alternately the (0.5kbp) GALI0 promoter (Schultz et al., I987, Gene, 54, ppII3-I23) was substituted for the I.Ikbp GAP promoter in the above construction, and the (I.25 kbp) ADH2 promoter (Kniskern et al., I988 Hepatology 8, 82-87) was substituted for the GAP promoter (see Figure I).

In each case, the expression cassette containing the specific promoter, the variant HBsAg ORF, and the ADHI terminator was cloned into the shuttle vector pCI/I (Beggs, supra; Rosenberg, et al., supra) to create a yeast expression vector which was then used to transform S. cerevisiae as described below.

EXAMPLE 5

Construction of yeast S. CerevisiaeCF52 (mnn9-) mutant yeast strain

Yeast S. cerevisiae strain KHY I07 (cir⁺, adeI⁺, leu2⁻ and mnn9-) was constructed as follows: The α mating type strain CZ5/LB347-IC (mnn9⁻, SUCZ⁻) was mated with the a type strain 2I50-2-3 (leu2⁻, adeI⁻) by mixing the strains on a YEHD complete media plate. To select for diploids, the mated strains were replica plated onto leu⁻ minimal medium containing 2% sucrose as the sole carbon source. After isolating single colonies, the diploids were sporulated, and asci were dissected by standard techniques. The KHY-I07 strain was isolated as a single spore and characterized as cir⁺, adeI⁺, leu2⁻, and mnn9⁻ (by Schiff stain technique).

KHYI07 (cir 0) was derived from strain KHYI07 (cir⁺) as described by Broach [Methods in Enzymology, I0I, Part C, pp 307-325, (I983)]. The cured strain was made ura3⁻ by integrating a disrupted ura3 gene. The resulting strain, KHY-I07ura3Δ, was grown in rich media to allow the accumulation of spontaneous mutations and a canavanine resistant mutant was selected. The mutant strain, CF55, was shown by complementation tests to be canI⁻. The GALI0pGAL4 expression cassette was integrated into the HIS3 gene of CF55 (Methods in Enzymology, I990, I85 pp297-309) to yield the final host strain CF52 (Mata leu2-2,II2 ura3Δ canI is3Δ::GALI0pGAL4-URA3, cir°).

EXAMPLE 6

Yeast Transformation and Seed Establishment for HBsAg in CF52 mnn9⁻ Mutant Yeast

The pCI/I pGALI0-vHBsAg-tADH-I plasmid described in Example 4 was used to transform S. cerevisiae strain CF52. Clones were selected on minimal medium (leu- containing IM sorbitol), established as frozen stocks (in I7% glycerol) and evaluated as described below.

EXAMPLE 7

Growth and Expression of the HBsAg Gene behind the GALI0 promoter in Yeast CF52 (mnn9⁻)

Clones of yeast containing the expression plasmid described in Example 6 above were plated onto leu⁻ selective agar plates containing IM sorbitol and incubated at 30°C for 2-3 days. These yeast were inoculated with 5-7 mL of complex YEHDS (YEHD + IM sorbitol) and the cultures were incubated at 30°C with aeration for I2-I8 hours. Flasks containing 50 mL YEHDS + 2% galactose media were inoculated from the above culture

14

(to an initial $A_{600}$ = 0.l) and incubated at 30°C with shaking (350 rpm) for 72 hours to a final $A_{600}$ of l0-l6. Samples of l0 $A_{600}$ units were dispensed into tubes, and the yeast cells were pelleted at 2,000 x g for l0 minutes. The pellets either were assayed directly or stored at -70°C for future assay. At the time of assay, the pellets were resuspended in 0.4 mL of phosphate-buffered saline containing 2mM PMSF. Yeast cells were broken by: l) the addition of 200-300 mg of washed glass beads (0.45 mm), 2) agitation on a vortex mixer for l5 minutes, 3) addition of TritonX-l00 to 0.5% (v/v), 4) agitation on a vortex mixer for 2 minutes, and 5) incubation at 4°C for l0-l5 minutes. Cellular debris and glass beads were removed by centrifugation at l3,000 x g for l0 minutes. The clarified supernatant fluid was removed and analyzed for protein [by the method of Lowry et al., J. Biol. Chem., l93, 265 (l95l)] and for HBsAg by (AUSRIA^R) assay (Abbott).

Typical assay results are shown below.

## TABLE I

| SEED DESCRITPION | AUSRIA, UG/ML | SP. ACT. UG/MG PROTEIN | P24 LEVEL IMMUNOBLOT |
|---|---|---|---|
| pGAL10 | 2.0, 1.3, 0.6 | 0.34, 0.20, 0.11 | +++ |
| pADH2 | 1.7, 1.0, 1.0 | 0.25, 0.18, 0.16 | +++ |
| non-variant S pGAL10 (adw) | 4.5 | 0.61 | + |

## EXAMPLE 8

Large Scale Growth of S. cerevisiae(mnn9⁻) Producing HBsAg in Fermentors

The frozen recombinant yeast culture was inoculated onto leu⁻ plates containing lM sorbitol. The plates were incubated inverted at 28°C for 2-3 days. The growth on the plates was resuspended in YEHDS and the resuspended growth was transferred into 2-L Erlenmeyer flask containing 500 mL of YEHDS, and 2% galactose. The flask was incubated at 28°C and 350 rpm in a controlled environment shaker incubator for l8-22 hours. These seed cultures then were used to inoculate the production stage vessels.

An inoculum (l-5% v/v) from one or more flasks was transferred into l6-L or 250-L fermentors containing l0-L or 200-L of YEHDS, respectively. The l6-L fermentors were operated at 500 rpm, 5 L/min air, and 28°C. The 250-L fermentors were operated at l60 RPM, 60 L/min air and 28°C. The fermentors were harvested 40-46 hrs after inoculation with the seed culture. Optical density values of l5.0 $A_{660}$ units typically were obtained. Harvesting consisted of concentrating the cells using a hollow fiber filtering device followed by washing the cells in buffered salt solutions. Cell slurries were assayed as described below or stored frozen at -70°C for further processing and analysis.

Small samples (0.6 mL) of 20% washed cell slurries were broken using glass beads (0.45-0.52 mm) in l.5-mL Eppendorf tubes. PMSF (6.5 μl of 200 mM stock) was added as a protease inhibitor. Aliquots were removed from the tubes after breakage and frozen at -70°C for immunoblot analysis. TRITON X-l00 was added to the remaining sample in the tubes to a final concentration of 0.5%, and the samples were mixed briefly and incubated at 4°C for 20-40 min. The cell debris was removed by centrifugation and the clarified cell extract assayed for antigen (Ausria^R) and protein (Lowry).

## EXAMPLE 9

Large Scale Purification of Recombinant HBsAg

About 250g of frozen cell paste (producing recombinant S protein) was resuspended to l7% wet

weight/volume (about 1500 ml) in phosphate buffered saline solution (PBS). The cells were heated to 45°C by immersion in a water bath. The cells were held at 45°C for 15 minutes and then cooled on ice to about 10°C. The cells were then disrupted by two passages through a Gaulin homogenizer.

Following homogenization, 10% Triton X-100 was added to a final concentration of 0.3% and mixed for about 15 minutes. The cell extract was then centrifuged at 3,600 x g for 20 minutes at 4°C, and the supernatant was collected.

The superantant was then passaged over a column containing about 200g of XAD-2 resin to remove the Triton X-100. The effluent was then passaged directly over a column containing about 150g of wide pore silica with a pore size of about 1,500 angstrom and a particle size of about 50 microns. The columns used were 5 cm diameter (Pharmacia) and were run at a flow rate of about 200 ml per hour.

The silica column was washed with PBS until the $A_{280}$ returned to baseline.

The S protein was eluted from the silica column using first, cold borate buffer (50 mM, pH 8.7, 4°C) at a flow rate of about 500 ml per hour, until a rise in the $A_{280}$ was observed. Once the $A_{280}$ began to rise the column was heated to 55°C and 55°C borate buffer was run through the column at about 500 ml per hour. The eluate containing S protein (about 1L) was collected on ice. The eluate was then concentrated to about 200 ml by difiltration against 50 mM borate buffer at pH 8.7, using a hollow fiber diafiltration unit with a molecular weight cutoff of $10^5$. The S protein was then filtered through a 0.2 micron filter and stored. The product was found to be stable with no significant degradation observed on Western blot analysis.


## Claims

1. An immunologic a epitope variant of hepatitis B virus surface protein which forms immunologically active particles.

2. The hepatitis B virus surface protein of Claim I with substantially reduced entrapped carbohydrate or glycoprotein content.

3. The hepatitis B virus surface protein according to Claim 2 produced in recombinant yeast cells which are genetically deficient for protein glycosylation.

4. The hepatitis B virus surface protein according to Claim 3 wherein the genetic deficiency of the yeast cells is in the mnn9 gene.

5. The hepatitis B virus surface protein of Claim 2 wherein the carbohydrate to protein ratio of the purified surface protein is less than 0.5.

6. The hepatitis B virus surface protein according to Claim I wherein the immunologic variant a epitope results from amino acid modification, substitution, deletion, or addition between amino acids I24 and I47 of the hepatitis B virus surface protein.

20

7. The hepatitis B virus surface protein according to Claim 6 with an amino acid substitution at position l45 of hepatitis B virus surface antigen.

8. The hepatitis B virus surface protein according to Claim 7 wherein the amino acid substitution at position l45 is to arginine from glycine.

9. A vaccine against hepatitis B virus for use in humans which comprises an immunologic a epitope variant of hepatitis B virus surface protein which forms particles with substantially reduced entrapped carbohydrate or glycoprotein content.

10. The vaccine according to Claim 9 wherein the immunologic variant a epitope results from amino acid modification, substition, deletion or addition between amino acids l24 and l47 of the hepatitis B virus surface protein.

11. The vaccine according to Claim l0 with an amino acid substitution at position l45 of hepatitis B virus surface protein.

12. The vaccine according to Claim ll wherein the amino acid substition at position l45 is to arginine from glycine.

13. The vaccine according to Claim 9 produced in recombinant yeast cells which are genetically deficient for protein glycosylation.

14. The vaccine according to Claim l3 wherein the genetic deficiency is in the <u>mnn</u>9 gene.

15. The vaccine according to Claim 9 wherein the carbohydrate to protein ratio of the surface protein is less than 0.5.

16. An immunologic diagnostic reagent which comprises an immunologic variant a epitope hepatitis B virus protein which forms particles with substantially reduced entrapped host cell carbohydrate or glycoproteins content, with reduced reactivity with naturally occurring anti-yeast antibodies.

17. The immunologic diagnostic reagent of Claim l6 wherein the carbohydrate to protein ratio of the purified surface protein is less than 0.5.

18. The immunologic diagnostic reagent of Claim 17 wherein the hepatitis B virus surface protein contains an amino acid substitution at position l45, to arginine from glycine.

FIG. 1

EP 0 511 855 A1

European Patent Office

**EUROPEAN SEARCH REPORT**

Application Number

EP 92 30 3882

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,P | WO-A-9 114 703 (IMPERIAL COLLEGE)<br>* the whole document * | 1,6-8 | C12N15/51<br>A61K39/29<br>G01N33/576<br>C07K15/00 |
| Y | EP-A-0 344 864 (MERCK AND CO. INC.)<br>* the whole document * | 2-5,9-18 | |
| Y | THE LANCET<br>vol. 336, 1990,<br>pages 325 - 329;<br>W. F. CARMAN ET AL.: 'Vaccine induced escape mutant of hepatitis B virus'<br>* the whole document * | 2-5,9-18 | |
| | | | **TECHNICAL FIELDS SEARCHED (Int. Cl.5)**<br><br>C07K<br>C12N<br>A61K<br>G01N |

The present search report has been drawn up for all claims

| Place of search | Date of completion of the search | Examiner |
|---|---|---|
| THE HAGUE | 13 AUGUST 1992 | SKELLY J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

23

SEQUENCE LISTING


(2) INFORMATION FOR SEQ ID NO:1:


(i) SEQUENCE CHARACTERISTICS:

(A) LENGTH: 10 base pairs

(B) TYPE: nucleic acid

(C) STRANDEDNESS: single

(D) TOPOLOGY: linear


(ii) MOLECULE TYPE: DNA (genomic)


(xi) SEQUENCE DESCRIPTION: SEQ ID NO:1:


ACAAAACAAA                                    10


(2) INFORMATION FOR SEQ ID NO:2:


(i) SEQUENCE CHARACTERISTICS:

(A) LENGTH: 30 base pairs

(B) TYPE: nucleic acid

(C) STRANDEDNESS: single

(D) TOPOLOGY: linear

(ii) MOLECULE TYPE: DNA (genomic)

(xi) SEQUENCE DESCRIPTION: SEQ ID NO:2:

AATTCAAGCT TACAAAACAA AATGCAGTGG                                    30

(2) INFORMATION FOR SEQ ID NO:3:

    (i) SEQUENCE CHARACTERISTICS:

        (A) LENGTH: 30 base pairs

        (B) TYPE: nucleic acid

        (C) STRANDEDNESS: single

        (D) TOPOLOGY: linear

    (ii) MOLECULE TYPE: DNA (genomic)

    (xi) SEQUENCE DESCRIPTION: SEQ ID NO:3:

GTTCGAATGT TTTGTTTTAC GTCACCTTAA                                    30

(2) INFORMATION FOR SEQ ID NO:4:

    (i) SEQUENCE CHARACTERISTICS:

        (A) LENGTH: 15 base pairs

        (B) TYPE: nucleic acid

        (C) STRANDEDNESS: single

        (D) TOPOLOGY: linear

(ii) MOLECULE TYPE: DNA (genomic)

(xi) SEQUENCE DESCRIPTION: SEQ ID NO:4:

ATACATTTAA AGCTTG                                                16

(2) INFORMATION FOR SEQ ID NO:5:

    (i) SEQUENCE CHARACTERISTICS:

        (A) LENGTH: 18 base pairs

        (B) TYPE: nucleic acid

        (C) STRANDEDNESS: single

        (D) TOPOLOGY: linear

    (ii) MOLECULE TYPE: DNA (genomic)

    (xi) SEQUENCE DESCRIPTION: SEQ ID NO:5:

TGTAAATTTC GAACCTAG                                               18

(2) INFORMATION FOR SEQ ID NO:6:

    (i) SEQUENCE CHARACTERISTICS:

        (A) LENGTH: 45 base pairs

        (B) TYPE: nucleic acid

        (C) STRANDEDNESS: single

        (D) TOPOLOGY: linear

(ii) MOLECULE TYPE: DNA (genomic)

(xi) SEQUENCE DESCRIPTION: SEQ ID NO:6:

AATTCAAGCT TACAAAACAA AATGGAGAAC ATCACATCAG GATTC                    45

(2) INFORMATION FOR SEQ ID NO:7:

    (i) SEQUENCE CHARACTERISTICS:

        (A) LENGTH: 45 base pairs

        (B) TYPE: nucleic acid

        (C) STRANDEDNESS: single

        (D) TOPOLOGY: linear

    (ii) MOLECULE TYPE: DNA (genomic)

    (xi) SEQUENCE DESCRIPTION: SEQ ID NO:7:

GTTCGAATGT TTTGTTTTAC CTCTTGTAGT GTAGTCCTAA GGATC                    45

(2) INFORMATION FOR SEQ ID NO:8:

    (i) SEQUENCE CHARACTERISTICS:

        (A) LENGTH: 40 base pairs

        (B) TYPE: nucleic acid

        (C) STRANDEDNESS: single

        (D) TOPOLOGY: linear

(ii) MOLECULE TYPE: DNA (genomic)

(xi) SEQUENCE DESCRIPTION: SEQ ID NO:8:

CCAGGACGAT GGGATGGGAA TACAGGTGCA ATTTCGATCC                    40

(2) INFORMATION FOR SEQ ID NO:9:

      (i) SEQUENCE CHARACTERISTICS:
          (A) LENGTH: 27 base pairs
          (B) TYPE: nucleic acid
          (C) STRANDEDNESS: single
          (D) TOPOLOGY: linear

      (ii) MOLECULE TYPE: DNA (genomic)

(xi) SEQUENCE DESCRIPTION: SEQ ID NO:9:

AGGATTCCTA GGACCCCTGC TCGTGTT                                  27